Europäisches Patentamt

European Patent Office

Office européen des brevets

(11) Publication number: **0 220 153 B1**

(12) **EUROPEAN PATENT SPECIFICATION**

(45) Date of publication of patent specification: 24.07.91   (51) Int. Cl.⁵: **A61K 31/02**, A61K 31/23, A61K 9/10, C12N 5/00, A01N 1/02

(21) Application number: 86850373.1

(22) Date of filing: 23.10.86

(54) **A nutritive emulsion having oxygen-transporting properties, and process for its preparation.**

(30) Priority: 25.10.85 SE 8505048

(43) Date of publication of application:
29.04.87 Bulletin 87/18

(45) Publication of the grant of the patent:
24.07.91 Bulletin 91/30

(84) Designated Contracting States:
DE ES FR GB IT SE

(56) References cited:
DE-B- 2 406 621       GB-A- 2 091 098
US-A- 3 793 450       US-A- 3 962 439
US-A- 4 252 827       US-A- 4 423 077
US-A- 4 446 154

(73) Proprietor: Kabi Pharmacia AB

S-751 82 Uppsala(SE)

(72) Inventor: Wretlind, Arvid
Floragatan 2
S-114 31 Stockholm(SE)
Inventor: Ljungberg, Stellan
Parksätravägen 19
S-181 61 Lidingö(SE)
Inventor: Hakansson, Ivan
Fleminggatan 59
S-112 32 Stockholm(SE)
Inventor: Jeppsson, Roland
S:t Eriksgatan 19
S-112 39 Stockholm(SE)
Inventor: Ajaxon, Bengt
7 Hävelvägen
S-752 47 Uppsala(SE)

(74) Representative: Hansson, Sven A. et al
AB STOCKHOLMS PATENTBYRA Box 3129
S-103 62 Stockholm(SE)

## Description

This invention relates to an emulsion having oxygen transporting and nutritive properties. Such an emulsion is suitable for use for oxygen transport as a nutrient solution in tissue culture and for perfusion of isolated organs, such as at a transplantation. The emulsion can also be used as a blood substitute with nutritive properties for humans and animals, when it is administrered intravenously.

The invention also refers to a process for preparing the above emulsion.

Oxygen transporting emulsions are previously known, e.g. from Swedish patents 403433, 415143, Swedish applications 76077270, 8300451-5, US patents 3 958 014, 3 962 439, 4 105 798, 4 252 827, 4 425 347, 4 446 154, 4 446 155, 4 186 253, 4 423 077, GB 1 409 944, 1 517 024, WO 84/00686, EP 807 16 and others. These emulsions consist substantially of fine particles of perfluorocarbon compounds which have been emulsified in an aqueous medium by the aid of various emulsifiers, such as phospholipides, fatty acids and various derivatives thereof. These emulsions have been found to have the desired properties with regard to an oxygen transporting ability.

. However, they suffer from a number of shortcomings. The most serious one is an inferior or bad stability, which principally appears if one tries to sterilize the emulsion in conventional manner by autoclaving at about 120°C. The particles will then lump together to aggregates which have too great dimensions to be infused into the blood vessels without risks. For this reason one has in practice been compelled to content oneself with a repeated pasteurization at about 60°C, and it is self-evident that this treatment will not give an equally high safety against microorganisms as a sterilization.

It has now surprisingly been found that by the addition of fats of animal and/or vegetable origin (e.g. fish oil, soybean oil, coconut oil or cottonseed oil) or triglycerides of a similar chemical composition, and small amounts of certain amino acids, the emulsions will be sufficiently stable to be sterilized by conventional autoclaving without formation of large particle aggregates. This is quite unexpected, since oil and perfluorocarbon compounds do not dissolve in one another. A certain amount of biologically useful energy is added to the emulsion as well, which is of great value when the emulsion is given to patients requiring an intravenous supply of nutrients. Furthermore, when the emulsion is used as an oxygen transporting vehicle in vitro (in tissue culture), a simultaneous supply of oxygen and biologically useful energy is of a great importance to cover the energy requirements of the tissues. This object is achieved with the emulsion of the present invention.

The included amount of fat can vary between 1 and 50 g per 100 ml of emulsion, preferably between 3 and 30 g per 100 ml.

The amino acids of interest in this respect include glycine, serine and phenylalanine. The amounts are relatively small, from 0.05 up to 2 g per 100 ml, preferably 0.05-0.8 g per 100 ml.

Thus, the present invention refers to an oxygen transporting, autoclavable nutrition emulsion, containing in a physiologically acceptable aqueous medium

a) from 5 to 50 g per 100 ml emulsion of one or more perfluorocarbon compounds, and

b) from 0.2 to 10 g per 100 ml of emulsion of one or more phospholipides as emulsifier. The invention is characterized in that the emulsion also contains

c) from 1 to 50 g per 100 ml of emulsion of animal or vegetable fat or triglycerides of a similar chemical composition, and

d) from 0.05 to 2 g per 100 ml of emulsion of one or more amino acids, with the proviso that at least one of said amino acids is glycine, serine or phenylalanine.

DE-B-2 406 621 relates to an amino acid-containing fat emulsion which is characterised by a particle size below 0.5 µm and a content of not more than 3 g of a fatty acid (or a basic amino acid salt thereof) having 12-20 carbon atoms, per gram of phospholipide.

GB-A-2 091 098 refers to an O/W emulsion for nutritive purposes, wherein maltose simultaneously serves the functions as an isotonic agent, an energy source and an emulsion stabilizer.

US-A-3 793 450 refers to amino acid-containing fat emulsions wherein the amino acids are present as free bases.

US-A-3 962 439 refers to oxygen-transporting fluorocarbon emulsions, which contain phospholipides as emulsifier and potassium palmitate or sodium oleate as contributing emulsifiers.

US-A-4 252 827 gives examples of the development within the field of fluorocarbon emulsions in its text, and discloses i.a. that emulsions having good excretion properties cannot be mixed with certain types of plasma expanders, as is sometimes desirable. This disadvantage has been remedied by the addition of a high-molecular-weight nonionic contributing emulsifier, and by having the fluorocarbon portion consist of a mixture of perfluorodecalin and perfluorotripropylamine.

These two patents give a good overview of the state of the art within this field, but they say nothing

2

about any combination of an oil and a small amount of an amino acid selected from a specific group of such acids.

US-A-4 423 077 refers to fluorocarbon emulsions wherein the particles of the fluorocarbon compounds have been provided with a lipid layer on their surfaces.

US-A-4 446 154 refers to a system for the treatment of acute cerebral injuries and is based on an addition of thereapeutic solutions directly into the cerebrospinal room. These solutions have been formulated to correspond to the composition of the cerebrospinal fluid, but with a fluorocarbon compound as an oxygen transporter.

The oxygen transporting component of the emulsion consists of an aliphatic perfluorocarbon compound which has 9-12 carbon atoms and may contain an alicyclic ring or a heterocyclic ring with a nitrogen or oxygen atom. Specifically, the perfluorocarbon compound may consist of a perfluorocycloalkane or perfluoroalkylcycloalkane, a perfluoro saturated heterocyclic compound with the hetero atoms nitrogen and/or oxygen, a perfluorinated tertiary amine, or a perfluorooxaalkane. These compounds have boiling points between about 140 and 160° C and have the ability of transporting oxygen amounting to at least 30 % by volume based on the compound. Examples of suitable types of perfluorocarbon compounds are given below.

The first group of perfluorocarbon compounds used, complying with the invention, comprises perfluorocycloalkanes or perfluoro(alkylcycloalkanes), such as perfluoro($C_{3-5}$-alkylcyclohexanes), such as perfluoro(methyl-propylcyclohexanes), perfluoro(butylcyclohexanes), percyclohexanes), perfluoro(pentylcyclohexanes), percyclohexanes) and perfluoro(pentylcyclohexanes); perfluorodecalin, and perfluoro(methyldecalins).

The second group comprises perfluoro (alkyl saturated heterocyclic compounds), such as perfluoro(alkyltetrahydropyranes), such as perfluoro(butyltetrahydropyranes), perfluoro(pentyltetrahydropyranes) and perfluoro(hexyltetrahydropyranes); perfluoro(alkyltetrahydrofuranes) such as perfluoro(pentyltetrahydrofuranes), perfluoro(hexyltetrahydrofuranes) and perfluoro(heptyltetrahydrofuranes); perfluoro(N-alkylpiperidines) such as perfluoro(N-pentylpiperidines), perfluoro(N-hexylpiperidines) and perfluoro(N-butylpiperidines); and perfluoro(N-alkylmorpholines), such as perfluoro(N-pentylmorpholines), perfluoro(N-hexylmorpholines) and perfluoro(N-heptylmorpholines).

The third group includes perfluoro(tert.-amines), e.g. perfluoro(diethylhexyl amines), perfluoro(dipropylbutyl amines) and perefluoro(diethylcyclohexyl amines); and perfluoro(dioxa-alkanes), that is perfluoro(alkyleneglycol dialkyl ether), such as perfluoro(3,8-dioxa-2,9-dimethyldecane), or perfluoro(tetramethylene glycol diisopropylether), perfluoro(3,7-dioxa-2,8-dimethylnonane) or perfluoro(trimethyleneglycol diisopropylether) and perfluoro(4,6-dioxa-5,5-dimethylnonane) or perfluoro(isopropylene glycolidine-propyl ether).

These perfluorocarbon compounds are used alone or as an isomer mixture; furthermore, two or more types of compounds can be mixed.

Compounds to be preferred among those mentioned above are the ones that are rapidly eliminated from the body, like perfluorodecalin and perfluoromethyldecalin.

The perfluorocarbon compounds are available commercially or can be prepared by methods known from the literature.

The perfluorocarbon compound can be included in the emulsion in an amount of 5-50 g per 100 ml of emulsion, most preferably 20-30 g per 100 ml. It goes without saying that a plurality of different perfluorocarbon compounds can be jointly included in a total amount within the indicated limits.

One or more emulsifiers are required to obtain a stable emulsion. Natural phospholipides have been found to be very suitable. These phospholipides or phosphatides can be derived from for instance eggs or soybeans. Natural egg phospholipides have turned out to be most suitable, since they are practically free from secondary effects following intravenous administration. The phospholipide is included in the emulsion in an amount of from 0.2 to 10 g per 100 ml of emulsion, preferably 1 to 8 g per 100 ml.

Other contributing emulsifiers ("coemulsifiers") of natural or synthetic origin can also be included in the emulsion to improve its stability. Of course such emulsifiers must be pharmacologically acceptable, and may consist of for instance cholesterol, cholesterol esters, polyoxyethylene-polyoxypropylene glycols, polyglycerol monooleate and the like, and also of mono- and diglycerides or alkylated glycerides, of which a number of various types are found on the market under various trade names. Synthetic emulsifiers may be included in an amount of up to 10 g per 100 ml of emulsion, preferably 0.2 to 8 g per 100 ml.

One or more monoglycerides of fatty acids having 8-22 carbon atoms, preferably 14-20 carbon atoms, may further be included in the emulsion. Examples of such acids are caprylic acid, capric acid, lauric acid, myristic acid, palmitic acid, stearic acid, behenic acid, palmitoleic acid, oleic acid, linoleic acid and arachidonic acid. Such monoglycerides can be included in the emulsion in an amount of 0.5 to 10 g per 100 ml of emulsion, preferably about 8 g per 100 ml. Fatty acid as such can also be included in the form of a salt, e.g. a sodium salt, and in an amount of 0.1 to 5 g, preferably 0.1 to 3 g, per 100 ml.

Besides the components mentioned above, the emulsions according to the invention may contain such constituents as preservatives, pH adjusting agents and agents for adjusting the osmotic pressure. An important group of additives consists of agents for making the emulsion isotonic with blood. Carbohydrates, such as glucose and fructose, or sugar alcohols, such as sorbitol, have been used for this purpose for a long time in nutrition solutions. However, the triol glycerol is preferred here since it also contributes to the stability of the emulsion. Furthermore, glycerol has since long been used as an isotonic agent which gives a significant addition of energy. In order to give the emulsion stabilizing and nutritive properties it must contain fat, which can be supplemented with various amino acids to widen the nutritional properties; concomitantly they increase the pH stability of the emulsion.

In a suitable emulsion complying with the invention, the particle size is restricted by the size which enables the particles, as well as the blood corpuscles (of a diameter of about 8μm) to pass through the fine capillaries of the body. It has been found advantageous to keep the particle size of the perfluorocarbon compounds less than 4 μm. However, the main portion should have a diameter well below 1μm, preferably between 0.05 and 0.5 μm. At this particle size, there seems to be a smaller tendency for accumulation in certain organs of the body, such as the spleen and the liver.

Certain perfluorocarbon compounds such as perfluorodecalin are eliminated through the lungs. In the emulsions according to the invention, there is no risk that the particles of the emulsion will lump into big aggregates at storage or sterilization. There is no accumulation risk, either, for the fat. In the preparation of emulsions according to the invention, a particle size is obtained which is less than that of the chylomicrons in the blood vessels.

In the preparation of an emulsion according to the invention, soybean oil, perfluorocarbon compound, various emulsifiers, fatty acid glyceride, amino acid and other optional components are homogeneously mixed in a physiologically acceptable aqueous medium by means of a suitable mixer, subsequently the pH is adjusted to a suitable value between 5 and 10, and the complete mixture is homogenized in a homogenizer. The components can first be blended in a conventional mixer, e.g. a propeller agitator. The homogenization of the mixture can be completed in a high-pressure homogenizer in which the mixture is forced through a slit at a temperature of 30-90°C, at high pressure and at high speed, whereby the mixture will be vigorously sheared and blended. Homogenizers of this type are commercially available and are sold e.g. by Manton-Gaulin Manufacturing Co, Ind., USA. In such a homogenizer the mixture is forced through the slit several times at a pressure in the order of 10 to 55 MPa whereby a stable emulsion according to the invention is obtained. After the preparation, the emulsion can be filled into suitable storage bottles. The emulsion is sterilized by autoclaving at 100°C-140°C for a period adapted to the autoclaving temperature. Following this treatment, the emulsion is still stable. It should be noted as remarkable and extremely unexpected that in the preparation of emulsions according to the invention, no special measures such as centrifuging or decanting are necessary; no big particles remain to be removed from the homogenization process. Earlier, this has often been necessary in the preparation of emulsion of perfluorocarbon compounds.

It is important that all components that are used are of a pharmacologically acceptable quality from the beginning, i.e. are free from pyrogens. The quality of the individual constituents is maintained throughout the whole manufacturing processes. Thus, it is essential that the fat, the phospholipides and the monoglycerides are prepared and purified in a proper way, e.g. by means of suitable extraction and precipitation processes. They should be protected from contact with oxygen during manufacture and storage to avoid the formation of oxidation products which may cause adverse reactions. This is of special importance when the emulsion is intended to be injected intravenously. Preparation of pharmacologically acceptable egg phospholipides and soybean oil is described i.a. in US patent No. 3,169,094. An inert atmosphere (such as nitrogen gas) must also be used in preparing and bottling of emulsions according to the invention.

The invention is illustrated in more detail by the following examples:

Example 1

An emulsion was prepared from the following components:

4

| Purified soybean oil | 3 | g |
| Perfluorodecalin (FLUTEC[(R)] PP5 from ISC Chemicals Ltd) | 28 | g |
| Egg phospholipides | 5 | g |
| Glycerol | 2.5 | g |
| Distilled water to | 100 | ml |

The components were first premixed in a conventional mixer, then homogenized in a homogenizer and filled into infusion bottles of 100 ml which were sealed by a rubber stopper and a capsule in a customary manner. The emulsion was then autoclaved in an autoclave at 121°C for 20 minutes. After this procedure the emulsion was shake tested for up to 45 hours without any noticeable deterioration of the quality. The pH of the emulsion after the autoclaving was found to be 8.4.

Example 2

An emulsion of the same composition and prepared in the same way as in example 1 was tested on mice for acute toxicity. The result of the toxicity test on mice is shown in the following Table. Observation time was 72 hours.

| Perfluorodecalin g/kg | 25.2 | 30.0 | 30.7 |
| Dose of emulsion i.v. { ml/kg body weight | 90 | 107 | 128 |
| ml/20 g | 1.8 | 2.1 | 26 |
| Mortality | 0/19 | 16/25 | 14/14 |
| expressed in % | 0 | 64 | 100 |

It appears that the acute toxicity ($LD_{50}$) will be just below 30 g/kg for perfluorodecalin. Owing to the great volumes that were injected, it cannot be excluded that changes in liquid and electrolyte balance have affected the results and that, therefore, an examination of this kind not solely reflects the toxicity of the fluorocarbon compound; however, the indicated value of 30 g/kg is of such a magnitude that the preparation can be regarded as non-toxic.

Following intravenous injections into cats of single doses of up to 5 ml, no effect on the blood pressure could be observed. In previous studies, "anaphylactic reactions" were reported after injections of fluorocarbon emulsified with synthetic emulsifiers. No such reactions were observed when native egg phospholipides were used as emulsifiers.

Thus, the conclusion can be drawn that the emulsion according to the invention has no toxic effects at normal intravenous dose levels.

Example 3

| Perfluorodecalin (FLUTEC[(R)] PP5, from ISC Chemicals Ltd) | 28 | g |
| Purified soybean oil | 3 | g |
| Egg phospholipides | 1.2 | g |
| Glycerol | 2.5 | g |
| Distilled water to | 100 | ml |

The emulsion was prepared using the same procedure as in example 1. The emulsion was used for tests on rats and rabbits after addition of a standard solution ADDAMEL® (see below) and albumin to a concentration of 10 % and 5 %, respectively.

Rat: The mixture was infused into the dorsal tail vein at a constant rate of 0.32 ml/min. At the same time blood (0.32 ml/min) was drawn from the jugular vein. The tests were carried out under ether anaesthesia. After administration of 48-62 ml of emulsion per kg body weight, the animals were allowed to wake up and inhale pure oxygen gas for about 2 hours. The volume of drawn blood-emulsion mixture was 44-51 ml/kg. All the animals survived and did not show any symptoms of disease or discomfort even after a long observation period.

Rabbit: A drawn blood volume was replaced with the same type of emulsion mixture thas was used in the above tests on rats. In this test the perfluorodecalin emulsion without any addition was injected in the left jugular vein and ADDAMEL® and the albumin solutions in the right jugular vein. $pO_2$, $pCO_2$ and pH were recorded in the aorta. Up to 30 ml/kg of perfluorodecalin emulsion was infused. In all tests an increase in $pCO_2$ and a decrease of pH were noted as in normal anesthetized animals. $pO_2$ was maintained constant or increased with between 30 and 40 % during the administration of the emulsion. The animals inhaled oxygen gas (100 % and at atmospheric pressure) as long as the infusion lasted.

ADDAMEL® is a sterile stock solution of electrolytes and trace elements for addition to an infusion solution.

Declaration:

Calcium chloride 109.5 mg, magnesium chloride 30.42 mg, ferric chloride 1.35 mg, zinc chloride 0.27 mg, manganese chloride 0.79 mg, copper chloride 85 micrograms, sodium fluoride 0.21 mg, potassium iodide 17 micrograms, sorbitol 0.3 g, sodium hydroxide q.s. to pH 2.5, sterile water to 1 ml. ADDAMEL is a registered trade mark.

A bottle of 10 ml contains:

| | | | | | |
|---|---|---|---|---|---|
| $Ca^{2+}$ | 5 | mmol | $Cu^{2+}$ | 5 | μmol |
| $Mg^{2+}$ | 1.5 | mmol | $F-$ | 50 | μmol |
| $Fe^{3+}$ | 50 | μmol | $J-$ | 1 | μmol |
| $Zn^{2+}$ | 20 | μmol | $Cl-$ | 13.3 | mmol |
| $Mn^{2+}$ | 40 | μmol | | | |

Example 4

In order to test effects on the blood pressure when using fluorocarbon emulsions prepared from fats of different origin, emulsions were prepared having the following composition:

| | | |
|---|---|---|
| Fat | 10 | % |
| Perfluorodecalin (FLUTEC® PP5, from ISC Chemicals Ltd) | 28 | % |
| Egg phospholipides | 7 | % |
| Glycerol | 2.5 | % |
| Distilled water to | 100 | % |
| pH adjusted within the range | 7-10 | |

The following fats were tested: Cottonseed oil, safflower oil, rapeseed oil, olive oil, corn oil and almond oil. The emulsions were prepared according to the procedure in example 1, and were tested with regard to influence on the blood pressure of cats. 0.28 ml of emulsion per kg of body weight was injected. None of the emulsions prepared from the above-mentioned fats gave any effect with regard to the blood pressure of the test animals.

Example 5

| Perfluorodecalin (FLUTEC ② PP5, from ISC Chemicals Ltd) | 28 | g |
|---|---|---|
| Purified soybean oil | 10 | g |
| Egg phospholipides | 7 | g |
| Glycerol | 2.5 | g |
| Distilled water to | 100 | ml |
| pH adjusted within the range | 7-10 | |

The emulsion was prepared using the same procedure as in example 1, with the addition that the emulsion was filled under nitrogen atmosphere.

Example 6

| Perfluorodecalin (FLUTEC ② PP5, from ISC Chemicals Ltd) | 28 | g |
|---|---|---|
| Purified soybean oil | 3 | g |
| Egg phospholipides | 5 | g |
| Glycerol | 2.5 | g |
| Distilled water to | 100 | ml |
| pH adjusted within the range | 7-10 | |

The emulsion was prepared using the same procedure as in example 5. The emulsions prepared according to examples 5 and 6 were tested with regard to pH changes during storage at $+37°$ C, $+25°$ C and $+4°$ C - $+8°$ C for up to 90 days. Storage at $+4°$ C - $+8°$ C gave no or slight change in pH. Storage at $+25°$ C resulted in a moderate pH change (decrease), while a striking decrease in pH was noted at $+37°$ C. The change in the content of free fatty acids was studied in emulsions prepared according to example 6. It was found that emulsions stored at $+4°$ C - $+8°$ C (refrigerator temperature) and at $+25°$ C (room temperature) did not show any marked change of the contents of free fatty acids, while they increased considerably in the emulsion stored at $+37°$ C.

Example 7

7

Perfluorodecalin (FLUTEC® PP5,

from ISC Chemicals Ltd)                                    28    g

Purified soybean oil                                       10    g

Egg phospholipides                                          7    g

Glycerol                                                  2.5   g

Linoleic acid                                             0.2   g

Distilled water to                                        100   ml

pH was adjusted within the range                         7-10

The emulsion was prepared in the same way as in example 1. The emulsion did not show any changes in shake tests.

Example 8

Perfluorodecalin (FLUTEC® PP5,

from ISC Chemicals Ltd)                                    28    g

Purified soybean oil                                       10    g

Egg phospholipides                                        1.2   g

Glycerol                                                  2.5   g

Amino acid                  corresponding to               1    g

Distilled water to                                        100   ml

Emulsions with the declared composition were prepared with the amino acids arginine, proline, glycine, lysine and cysteine (the two last-mentioned were used in the form of hydrochlorides). pH was adjusted within the range of 7-8.5; preparation and autoclaving were carried out according to example 1.

The emulsions were shake tested for 2-20 hours and a series of emulsions with pH 8.5 were examined after 2, 4 and 6 hours while another series with pH 7.8 was examined after shaking for 10 and 20 hours. No particles of oil or perfluorodecalin were observed in either group. The pH did not show any appreciable change after autoclaving in any of the emulsions.

Example 9

Results of tests performed to determine the elimination of perfluorodecalin emulsions from the blood after i.v. injection.

Two different perfluordecalin (PFDC) emulsions were used in the investigations. These emulsions were composed as follows.

|  | A |  | B |  |
|---|---|---|---|---|
| PFDC | 28 | g | 28 | g |
| Soybean oil | 3 | g | – |  |
| Egg phospholipides | 5 | g | 5 | g |
| Glycerol | 2.5 | g | 2.5 | g |
| Distilled water to | 100 | ml | 100 | ml |
| pH | 8.4 |  | pH 8.0 |  |

The only difference between the emulsions A and B was the absence of soybean oil in B. The pH of the emulsions was determined directly after autoclaving.

The amount injected was 1 ml/kg. The elimination from the blood was determined nephelometrically on three rabbits for emulsion A and on two for emulsion B. The nephelometric determinations were carried out using previously described methods (Jeppson & Rössner, Acta pharmacol. et toxicol. 37, 134-144, 1975).

In this test, emulsion B gave no measurable light scattering values due to the weak scattering ability (confirmed in later tests). On the other hand, with emulsion A values of the same order of magnitude as in a soybean emulsion with the same oil content were obtained. The elimination from the blood was also the same, showing that the utilization degree of the oil in emulsion A had not been impaired by the presence of perfluorodecalin.

Example 10

| | | |
|---|---|---|
| Perfluorodecalin (FLUTEC® PP5, from ISC Chemicals Ltd) | 28 | g |
| Purified soybean oil | 10 | g |
| Egg phospholipides | 5 | g |
| Glycerol | 2.25 | g |
| Glycine | 0.25 | g |
| Sodium hydroxide solution 1 M | to pH 7-10 | |
| Sterile water | to 100 | ml |

An emulsion was prepared from the constituents and in the same way as in example 1. The emulsion was subjected to a shake test and was found to have the desired stability.

Example 11

Perfluorodecalin (FLUTEC® PP5,

from ISC Chemicals Ltd) 28 g

Purified soybean oil 10 g

Egg phospholipides 5 g

Glycerol 2.25 g

Serine 0.25 g

Sodium hydroxide solution 1 M to pH 7-10

Sterile water to 100 ml

An emulsion was prepared from the constituents in the same way as in example 1. The emulsion was tested by a shake test and was found to have the desired stability.

Example 12

Perfluorodecalin (FLUTEC® PP5,

from ISC Chemicals Ltd) 28 g

Purified soybean oil 10 g

Egg phospholipides 5 g

Glycerol 2.25 g

Phenylalanine 0.25 g

Sodium hydroxide solution, 1 M to pH 7-10

Sterile water to 100 ml

An emulsion was prepared from the above constituents and in the same way as in example 1. The emulsion was tested for stability by means of a shake test.

Example 13

In order to test the effect of the concentrations of the included substances on the stability of the emulsion, these were prepared with contents given in the specification in Table 1.

EP 0 220 153 B1

Example 14

### Table 1

|  | A | B | C | D | E | F | G | H | I |
|---|---|---|---|---|---|---|---|---|---|
| Perfluorodecalin, g (FLUTEC PP5) | 28 | 28 | 28 | 28 | 28 | 28 | 28 | 28 | 28 |
| Purified soybean oil, g | – | 3 | 10 | 3 | 10 | 10 | 10 | 10 | 10 |
| Egg phospholipides, g | 5 | 5 | 5 | 5 | 5 | 5 | 5 | 5 | 5 |
| Glycerol, g | 2.5 | 2.5 | 2.5 | 2.5 | 2.5 | 2.5 | 2.5 | 2.5 | 2.5 |
| Glycine, g | – | – | – | 0.25 | – | – | – | – | – |
| Serine, g | – | – | – | – | 0.25 | – | 0.1 | – | – |
| Phenylalanine, g | – | – | – | – | – | 0.25 | – | 0.1 | 0.05 |
| Sodium hydroxide solution to pH 7-10 | | | | | | | | | |
| Sterile water to (ml) | 100 | 100 | 100 | 100 | 100 | 100 | 100 | 100 | 100 |
| Shaking time in hours until the appearance of the emulsion is changed (upon visual inspection) | 24 | 72 | 24 | 96 | 96 | 96 | 96 | 96 | 96 |

| Perfluorotripropylamine (FC43) | 28 | g |
| Purified soybean oil | 3 | g |
| Egg phospholipides | 5 | g |
| Glycerol | 2.5 | g |
| Distilled water to | 100 | ml |
| pH was adjusted within the range | 7-10 | |

The emulsion was prepared using the same procedure as in example 1. pH after autoclaving was 6.60.

Example 15

| Perfluoromethyldecalin | 28 | g |
| Purified soybean oil | 3 | g |
| Egg pospholipides | 5 | g |
| Glycerol | 2.5 | g |
| Distilled water to | 100 | ml |
| pH was adjusted within the range | 7-10 | |

The emulsion was prepared using the same procedure as in example 1. The pH of the emulsion after autoclaving was 6.80. The emulsion did not show any change during shaking tests.

Example 16

| Perfluoromethyldecalin | 28 | g |
| Purified soybean oil | 10 | . |
| Egg phospholipides | 7 | |
| Glycerol | 2.5 | g |
| Distilled water to | 100 | ml |
| pH was adjusted within the range | 7-10 | |

The emulsion was prepared using the same procedure as in example 1. The pH of the emulsion after autoclaving was 6.90. The emulsion did not show any change during shaking tests.

Example 17

| | | |
|---|---|---|
| Perfluorodecalin | 28 | g |
| Myvacet ® (acetylated monoglycerides) | 3 | g |
| Purified soybean oil | 10 | g |
| Egg phospholipides | 5 | g |
| Glycerol | 2.25 | g |
| Phenylalanine | 0.1 | g |
| Sodium hydroxide solution, 1M | to pH 7.10 | |
| Sterile water | to 100 | ml |

An emulsion was prepared from the above constituents in the same way as in example 1. The stability of the emulsion was tested as in example 1. The appearance was unchanged after a shaking period of 96 hours.

Example 18

| | | |
|---|---|---|
| Perfluorodecalin | 28 | g |
| Miglyol ® 810 | 10 | g |
| Egg phospholipides | 5 | g |
| Glycerol | 2.25 | g |
| Phenylalanine | 0.1 | g |
| Sodium hydroxide solution 1 M | to pH 7-10 | |
| Sterile water | to 100 | ml |

Miglyol 810 is a registered trade mark for a triglyceride of fractionated coconut oil fatty acids $C_8$ - $C_{10}$, sold by Dynamit Nobel Chemicals.

An emulsion is prepared from the ingredients in the same way as in Example 1. The emulsion was tested for stability in the same way as in Example 1. Its appearance was unchanged after 96 hours of shaking.

**Claims**

1. An oxygen transporting, autoclavable nutrition emulsion, containing in a physiologically acceptable aqueous medium
   a) from 5 to 50 g per 100 ml of emulsion of one or more perfluorocarbon compounds, and
   b) from 0.2 to 10 g per 100 ml of emulsion of one or more phospholipides as emulsifier,
   **characterized** in that the emulsion also contains
   c) from 1 to 50 g per 100 ml of emulsion of animal or vegetable fat or triglycerides of a similar chemical composition, and
   d) from 0.05 to 2 g per 100 ml of emulsion of one or more amino acids, with the proviso that at least one of said amino acids is glycine, serine or phenylalanine.

2. Emulsion as claimed in claim 1,
   **characterized** in that the fat or triglycerides of a similar chemical composition are included in an amount from 3 to 30 g per 100 ml of emulsion.

3. Emulsion as claimed in claim 1 or 2,

EP 0 220 153 B1

characterized in that the phospholipides are included in an amount from 1 to 8 g per 100 ml of emulsion.

4. Emulsion as claimed in any one of claims 1-3,
characterized in that the phospholipides consist of synthetic phospholipides or native phospholipides prepared from soybeans or eggs.

5. Emulsion as claimed in any one of claims 1-4,
characterized in that the perfluorocarbon compound contains from 9 to 12 carbon atoms and also at least one alicyclic ring or a heterocyclic ring with oxygen and/or nitrogen as hetero atoms.

6. Emulsion as claimed in any one of claims 1-5,
characterized in that the perfluorocarbon compound is included in an amount of from 20 to 30 g per 100 ml of emulsion.

7. Emulsion as claimed in any one of claims 1-6,
characterized in that the amount of glycine, serine or phenylalanine is 0.05 to 0.8 g per 100 ml.

8. Emulsion as claimed in any one of claims 1-7,
characterized in that the included particles have a maximum size of 8 $\mu$m, preferably between 0.1 and 1 $\mu$m.

9. Emulsion as claimed in claim 8,
characterized in that the average diameter of the particles is less than 1 $\mu$m, and preferably less than 0.5 $\mu$m.

10. Emulsion as claimed in any one of claims 1-9,
characterized in that the perfluorocarbon compound consists of perfluorodecalin or perfluoromethyldecalin.

11. A process for the preparation of an emulsion according to any of claims 1-10, wherein
a) from 5 to 50 g per 100 ml of emulsion of one or more perfluorocarbon compounds, and
b) from 0.2 to 10 g per 100 ml of emulsion of one or more phospholipides as emulsifier, are dispersed in a physiologically acceptable, aqueous medium, after which the resulting mixture is homogenized,
characterized in that before the dispersion is also added
c) from 1 to 50 g per 100 ml of emulsion of animal or vegetable fat or triglycerides of a similar chemical composition, and
d) from 0.05 to 2 g par 100 ml of emulsion of one or more amino acids, with the proviso that at least one of said amino acids is glycine, sarine or phenylalanine.

12. The process as claimed in claim 11,
characterized in that the homogenization is carried out so far that the emulsion will have a particle size below 4 $\mu$m, preferably between 0.1 and 1 $\mu$m.

13. The process as claimed in claim 11 or 12,
characterized in that the emulsion is sterilized after preparation by autoclaving at 110 - 140° C for a time adapted to the autoclaving temperature, preferably at 121° C for 20 min.

**Revendications**

1. Emulsion nutritive capable de transporter l'oxygène et d'être mise en autoclave, et contenant dans un milieu aqueux physiologiquement compatible
a) de 5 à 50 g pour 100 ml d'émulsion d'un ou de plusieurs composés perfluorocarbonés, et
b) de 0,2 à 10 g pour 100 ml d'émulsion d'un ou de plusieurs phospholipides en tant qu'agent émulsifiant, caractérisée en ce que l'émulsion contient également
c) de 1 à 50 g pour 100 ml d'émulsion de matières grasses animales ou végétales ou de triglycérides d'une composition chimique similaire, et

14

d) de 0,05 à 2 g pour 100 ml d'émulsion d'un ou de plusieurs acides aminés, à condition qu'au moins l'un des acides aminés soit la glycine, la sérine ou la phénylalanine.

2. Emulsion selon la revendication 1,
caractérisée en ce que les matières grasses ou les triglycérides d'une composition chimique similaire sont incorporés dans une quantité allant de 3 à 30 g pour 100 ml d'émulsion.

3. Emulsion selon la revendication 1 ou 2,
caractérisée en ce que les phospholipides sont incorporés dans une quantité allant de 1 à 8 g pour 100 ml d'émulsion.

4. Emulsion selon l'une quelconque des revendications 1-3,
caractérisée en ce que les phospholipides consistent en des phospholipides synthétiques ou en des phospholipides naturels préparés à partir de soja ou d'oeuf.

5. Emulsion selon l'une quelconque des revendications 1-4,
caractérisée en ce que le composé perfluorocarboné contient de 9 à 12 atomes de carbone et également au moins un composé cyclique aliphatique ou un composé hétérocyclique avec de l'oxygène et/ou de l'azote en tant qu'hétéro-atomes.

6. Emulsion selon l'une quelconque des revendications 1-5,
caractérisée en ce que le composé perfluorocarboné est incorporé dans une quantité allant de 20 à 30 g pour 100 ml d'émulsion.

7. Emulsion selon l'une quelconque des revendications 1-6,
caractérisée en ce que la quantité de glycine, de sérine ou de phénylalanine est de 0,05 à 0,8 g pour 100 ml.

8. Emulsion selon l'une quelconque des revendications 1-7,
caractérisée en ce que les particules incorporées ont une granulométrie maximum de 8 $\mu$, de préférence entre 0,1 et 1 $\mu$.

9. Emulsion selon la revendication 8,
caractérisée en ce que le diamètre moyen des particules est inférieur à 1 $\mu$, et de préférence inférieur à 0,5 $\mu$.

10. Emulsion selon l'une quelconque de revendications 1-9,
caractérisée en ce que le composé perfluorocarboné est constitué par la perfluorodécaline ou la perfluorométhyldécaline.

11. Procédé pour la préparation d'une émulsion selon l'une quelconque des revendications 1-10, dans lequel
a) de 5 à 50 g pour 100 ml d'émulsion d'un ou de plusieurs composés perfluorocarbonés, et
b) de 0,2 à 10 g pour 100 ml d'émulsion d'un ou plusieurs phospholipides en tant qu'agent émulsifiant, sont mis en dispersion dans un milieu aqueux compatible physiologiquement, après quoi le mélange obtenu est homogénéisé,
caractérisé en ce qu'avant la mise en dispersion on ajoute également
c) de 1 à 50 g pour 100 ml d'émulsion de matières grasses animales ou végétales ou des triglycérides d'une composition chimique similaire, et
d) de 0,05 à 2 g pour 100 ml d'émulsion d'un ou plusieurs acides aminés, à condition qu'au moins l'un des acides aminés soit la glycine, la sérine ou la phénylalanine.

12. Procédé selon la revendication 11,
caractérisé en ce que l'homogénéisation est effectuée tant que l'émulsion présente des particules ayant une granulométrie inférieure à 4 $\mu$, de préférence entre 0,1 et 1 $\mu$.

13. Procédé selon la revendication 11 ou 12,
caractérisé en ce que l'émulsion est stabilisée après la préparation par mise en autoclave à 110-

EP 0 220 153 B1

140°C pendant une durée adaptée à la température du séjour en autoclave, de préférence à 121°C pendant 20 minutes.

**Patentansprüche**

1. Sauerstofftransportierende, autoklavierbare Nähremulsion, enthaltend in einem physiologisch verträglichen wäßrigen Medium
   a) 5 bis 50 g einer Perfluorkohlenstoffverbindung oder mehrerer pro 100 ml Emulsion und
   b) 0,2 bis 10 g eines Phospholipids oder mehrerer als Emulgator pro 100 ml Emulsion,
   dadurch gekennzeichnet, daß die Emulsion weiterhin
   c) 1 bis 50 g eines tierischen oder pflanzlichen Fettes oder Triglyceride mit einer ähnlichen chemischen Zusammensetzung pro 100 ml Emulsion und
   d) 0,05 bis 2 g einer Aminosäure oder mehrerer pro 100 ml Emulsion, mit der Bedingung, daß mindestens eine der Aminosäuren Glycin, Serin oder Phenylalanin ist,
   enthält.

2. Emulsion nach Anspruch 1, dadurch gekennzeichnet, daß das Fett bzw. die Triglyceride mit einer ähnlichen chemischen Zusammensetzung in einer Menge von 3 bis 30 g pro 100 ml Emulsion enthalten sind.

3. Emulsion nach Anspruch 1 oder 2, dadurch gekennzeichnet, daß die Phospholipide in einer Menge von 1 bis 8 g pro 100 ml Emulsion enthalten sind.

4. Emulsion nach einem jeden der Ansprüche 1 bis 3, dadurch gekennzeichnet, daß die Phospholipide aus synthetischen Phospholipiden oder nativen Phospholipiden, hergestellt aus Sojabohnen oder Eiern, bestehen.

5. Emulsion nach einem jeden der Ansprüche 1 bis 4, dadurch gekennzeichnet, daß die Perfluorkohlenstoffverbindung 9 bis 12 Kohlenstoffatome und weiterhin mindestens einen alicyclischen Ring oder heterocyclischen Ring mit Sauerstoff und/oder Stickstoff als Heteroatome enthält.

6. Emulsion nach einem jeden der Ansprüche 1 bis 5, dadurch gekennzeichnet, daß die Perfluorkohlenstoffverbindung in einer Menge von 20 bis 30 g pro 100 ml Emulsion enthalten ist.

7. Emulsion nach einem jeden der Ansprüche 1 bis 6, dadurch gekennzeichnet, daß die Menge an Glycin, Serin oder Phenylalanin 0,05 bis 0,8 g pro 100 ml beträgt.

8. Emulsion nach einem jeden der Ansprüche 1 bis 7, dadurch gekennzeichnet, daß die enthaltenen Teilchen eine maximale Größe von 8 $\mu$m, vorzugsweise zwischen 0,1 und 1 $\mu$m, aufweisen.

9. Emulsion nach Anspruch 8, dadurch gekennzeichnet, daß der durchschnittliche Durchmesser der Teilchen weniger als 1 um und vorzugsweise weniger als 0,5 $\mu$m beträgt.

10. Emulsion nach einem jeden der Ansprüche 1 bis 9, dadurch gekennzeichnet, daß die Perfluorkohlenstoffverbindung aus Perfluordecalin oder Perfluormethyldecalin besteht.

11. Verfahren zur Herstellung einer Emulsion gemäß einem jeden der Ansprüche 1 bis 10, bei dem
    a) 5 bis 50 g einer Perfluorkohlenstoffverbindung oder mehrerer pro 100 ml Emulsion und
    b) 0,2 bis 10 g eines Phospholipids oder mehrerer als Emulgator pro 100 ml Emulsion,
    in einem physiologisch verträglichen, wäßrigen Medium dispergiert werden, wonach das erhaltene Gemisch homogenisiert wird, dadurch gekennzeichnet, daß vor der Dispersion weiterhin
    c) 1 bis 50 g eines tierischen oder pflanzlichen Fettes oder Triglyceride mit einer ähnlichen chemischen Zusammensetzung pro 100 ml Emulsion und
    d) 0,05 bis 2 g einer Aminosäure oder mehrerer pro 100 ml Emulsion, mit der Bedingung, daß mindestens eine der Aminosäuren Glycin, Serin oder Phenylalanin ist,
    zugesetzt werden.

12. Verfahren nach Anspruch 11, dadurch gekennzeichnet, daß die Homogenisierung so weit geführt wird,

16

daß die Emulsion eine Teilchengröße unterhalb von 4 $\mu$m, vorzugsweise zwischen 0,1 und 1 $\mu$m, aufweist.

13. Verfahren nach Anspruch 11 oder 12, dadurch gekennzeichnet, daß die Emulsion nach der Herstellung durch Autoklavierung bei 110 bis 140°C für eine der Autöklavierungstemperatur angepaßte Zeitspanne, vorzugsweise bei 121°C für 20 Minuten, sterilisiert wird.